# EUROPEAN PATENT APPLICATION

(11) **EP 4 403 182 A1**
(43) Date of publication of application: **24.07.2024**
(21) Application number: 22870019.1
(22) Date of filing: 15.09.2022
(51) Int. Cl.: A61K 39/00, A61K 39/39, A61K 45/00, A61P 35/00, A61P 35/02, A61K 9/08, A61K 9/14, A61K 47/36, A61K 38/02, A61K 38/16, A61K 31/437, A61K 31/7088, A61K 31/715, A61K 31/716, A61K 35/74

(54) **MEDICAMENT FOR TREATMENT AND/OR PREVENTION OF CANCER**

(30) Priority: 16.09.2021 JP 2021150931
(71) Applicant: Toray Industries, Inc., Tokyo 103-8666 (JP)
(72) Inventor: SHIBAHARA Kyoko, Kamakura-shi, Kanagawa 248-8555 (JP); OKANO Fumiyoshi, Kamakura-shi, Kanagawa 248-8555 (JP)
(74) Representative: Mewburn Ellis LLP
(86) International application number: PCT/JP2022/034501
(87) International publication number: WO 2023/042872

(57) **Abstract**

A combination medicament comprising: a particle comprising an amphiphilic polymer in which a hydrophobic segment is poly(hydroxy acid) and a hydrophilic segment is polysaccharide, and a cancer antigen; and an immuno-stimulating factor, can be used so that the particle and the immuno-stimulating factor are separately administered to a subject, thereby allowing for enhancement in treatment and/or prevention of cancer effect.

## Description

### Technical Field

The present invention relates to a medicament for treatment and/or prevention of cancer characterized in that a cancer antigen and an immuno-stimulating factor are administered separately.

### Background Art

As methods for treating or preventing cancer, "cancer vaccines", administering cancer antigens (for example, proteins and/or their peptide fragments) to be specifically expressed for cancer cells and inducing immune reactions against cancer cells *in vivo,* have been attempted. Such cancer vaccines, unlike general low-molecule anticancer agents, allow only cancer cells to be attacked by immune cells and thus have few side effects, and the effect of preventing the reoccurrence of cancer can also be expected due to the establishment of immune memory in the body. However, only a few examples of cancer vaccines, such as "Provenge" (dendritic cell vaccine against prostate cancer) and "Imygic" (oncolytic virus formulation against metastatic melanoma), are on the market because the technical development is highly difficult.

While there is a demand for development of potent adjuvants in order to enhance immune responses to antigens, adjuvants to be applied to humans are currently limited due to safety concerns, and no substances reported to have a high adjuvant effect are approved. For example, there are aluminum hydroxide adjuvants, but such adjuvants are not sufficient in immune activation capacity and need to be repeatedly administered in order to acquire immunity.

Methods for enclosing antigens in particles have been attempted for new adjuvants to provide high immune activation capacity. The methods are expected to provide improved safety and enhanced delivery efficiency to target cells by forming a particle to enclose antigens. Particulate adjuvants with diverse materials such as fatty acids, biodegradable polymers, and viral proteins (Non Patent Literature 1 and 2) have been proposed. In recent years, a technique related to an antigen-adjuvant particle complex in which an antigen is enclosed in an adjuvant particle comprising an amphiphilic polymer in which a hydrophobic segment is poly(hydroxy acid) and a hydrophilic segment is polysaccharide has been reported (Patent Literature 1 and 2). In particular, Patent Literature 2 describes that by using an antigen expressed in cancer as a complex, the antitumor effect is exerted, and application as particulate adjuvants to therapeutic agents for cancer is expected.

Treatment methods with a combination of therapeutic agents for cancers are clinically used as standard treatment methods in order to enhance the effectiveness of the therapeutic agents for cancers. Patent Literature 1 discloses that mixing the above particulate adjuvant with an immuno-stimulating factor enhances an immune activation capacity.

### Citation List

### Patent Literature

Patent Literature 1: WO2010/098432
Patent Literature 2: WO2015/053354

### Non Patent Literature

Non Patent Literature 1: Immunology, vol. 117, 2006, pages 78 to 88
Non Patent Literature 2: Nature Materials, vol. 10, 2011, pages 243 to 251

### Summary of Invention

### Technical Problem

While combination of a particulate adjuvant and an immuno-stimulating factor is hopeful approach for cancer treatment and/or prevention methods, studies about an effective combination have been insufficient. An object of the present invention is thus to establish a technique for enhancing the cancer treatment/prevention effect of a medicament in which a particulate adjuvant and an immuno-stimulating factor are used in combination.

### Solution to Problem

In order to overcome the above problems, the present inventor has made intensive studies focusing on a particle comprising an amphiphilic polymer in which a hydrophobic segment is poly(hydroxy acid) and a hydrophilic segment is polysaccharide, and a cancer antigen (hereinafter, often referred to as "particle" in the present specification), which is a hopeful particulate adjuvant for treatment or prevention of cancer. As a result, it was found that the particle and an immuno-stimulating factor exert a remarkable antitumor effect when they are administered separately, and thus the present invention has been completed.

In other words, the present invention has the constitution as shown in the following (1) to (15).
(1) A medicament for treatment and/or prevention of cancer, characterized in that the medicament is used to separately administer a particle and an immuno-stimulating factor, wherein the particle comprises an amphiphilic polymer in which a hydrophobic segment is poly(hydroxy acid) and a hydrophilic segment is polysaccharide, and a cancer antigen.
   (1-1) A combination medicament for use in the treatment and/or prevention of cancer, comprising a particle and an immuno-stimulating factor, wherein the particle comprises an amphiphilic polymer in which a hydrophobic segment is poly(hydroxy acid) and a hydrophilic segment is polysaccharide, and a cancer antigen, wherein the use is characterized in that administration of the particle and administration of the immuno-stimulating factor are performed separately.
   (1-2) The medicament according to (1-1), wherein the use is characterized in that administration of the particle and administration of the immuno-stimulating factor are performed separately by administration methods different from each other.
(2) The medicament according to any of (1) to (1-2), wherein the immuno-stimulating factor is a ligand or agonist binding to a Toll-like receptor (TLR), a NOD-like receptor (NLR), a RIG-like receptor, a C-type lectin receptor (CLR) or a stimulator of interferon gene (STING).
(3) The medicament according to (2), wherein the ligand or agonist binding to the Toll-like receptor (TLR) is a ligand or agonist binding to TLR2, TLR3, TLR4, TLR5, TLR7, TLR8, TLR9 or TLR11.
(4) The medicament according to (3), wherein the ligand or agonist binding to the Toll-like receptor (TLR) is any of the following (i) to (vii):
   (i) a ligand or agonist binding to TLR2 selected from the group consisting of peptidoglycan, lipoprotein, lipopolysaccharide, and zymosan;
   (ii) a ligand or agonist binding to TLR3 selected from the group consisting of poly(I:C) and poly(A:U);
   (iii) a ligand or agonist binding to TLR4 selected from the group consisting of lipopolysaccharide (LPS), HSP60, RS09, and MPLA;
   (iv) flagellin as a ligand or agonist binding to TLR5;
   (v) a ligand or agonist binding to TLR7 or 8 selected from the group consisting of an imidazoquinoline compound and single-strand RNA;
   (vi) a ligand or agonist binding to TLR9 selected from the group consisting of bacterial DNA, unmethylated CpG DNA, hemozorin, ODN1585, ODN1668, and ODN1826; and
   (vii) a ligand or agonist binding to TLR11 selected from the group consisting of profilin and uropathogenic bacteria.
(5) The medicament according to (4), wherein the imidazoquinoline compound (imidazoquinoline species) is imiquimod.
(6) The medicament according to any of (1) to (5), characterized in that the immuno-stimulating factor is administered to a local tumor area.
(6-1) The medicament according to any of (1) to (5), wherein administration of the immuno-stimulating factor is administration to a local tumor area.
(7) The medicament according to any of (1) to (6-1), wherein the polysaccharide is dextran, β-glucan, mannan, chitin, chitosan, gellan gum, alginic acid, hyaluronic acid or pullulan.
(8) The medicament according to (7), wherein the β-glucan is a polymer of glucose linked by one or more β-1,3 bonds and/or one or more β-1,6 bonds.
(9) The medicament according to (7) or (8), wherein the β-glucan is black yeast glucan, curdlan, pachyman, laminaran, lichenan, schizophyllan, lentinan, scleroglucan or pachymaran.
(10) The medicament according to any of (1) to (9), wherein the poly(hydroxy acid) is poly(lactic-co-glycolic acid), polylactic acid, or polyglycolic acid.
(11) The medicament according to any of (1) to (10), wherein a number-average molecular weight of the amphiphilic polymer is 500 to 100,000.
(12) The medicament according to any of (1) to (11), wherein an average particle size of the particle is 0.1 to 50 µm.
(13) The medicament according to any of (1) to (12), wherein the cancer is basal cell cancer, Paget's disease, skin cancer, breast cancer, kidney cancer, pancreatic cancer, colon cancer, lung cancer, brain tumor, gastric cancer, uterus cancer, ovarian cancer, prostate cancer, urinary bladder cancer, esophageal cancer, leukemia, lymphoma, liver cancer, gallbladder cancer, sarcoma, mastocytoma, adrenal cortex cancer, Ewing's tumor, Hodgkin's lymphoma, mesothelioma, multiple myeloma, testicle cancer, thyroid cancer, or head and neck cancer.
(14) A method for treating and/or preventing cancer, comprising separately administering: a particle comprising an amphiphilic polymer in which a hydrophobic segment is poly(hydroxy acid) and a hydrophilic segment is polysaccharide, and a cancer antigen; and an immuno-stimulating factor.
(15) The method according to (14), wherein administration of the particle and administration of the immuno-stimulating factor are separately performed by administration methods different from each other.

The present specification encompasses the disclosure of Japanese Patent Application No. 2021-150931 which is the basis of the priority claim of the present application.

### Advantageous Effects of Invention

Using a particle and an immuno-stimulating factor according to the present invention to administer separately to the each other, wherein the particle comprises an amphiphilic polymer in which a hydrophobic segment is poly(hydroxy acid) and a hydrophilic segment is polysaccharide, and a cancer antigen exert a remarkable antitumor effect and is effective for treatment and/or prevention of cancer.

### Brief Description of Drawings

[Figure 1] Figure 1 represents the results of GPC measurement of dextran-NH₂ and dextran-PLGA.
[Figure 2] Figure 2 represents the results of ¹H-NMR measurement of dextran-PLGA.
[Figure 3] Figure 3 represents the results of GPC measurement of black yeast glucan-NH₂ and black yeast glucan-PLGA.
[Figure 4] Figure 4 represents the results of ¹H-NMR measurement of black yeast glucan-PLGA.
[Figure 5] Figure 5 represents the results of DLS measurement of an OVA-containing dextran particle.
[Figure 6] Figure 6 represents the results of DLS measurement of an OVA-containing black yeast glucan particle.

### Description of Embodiments

The present invention is characterized in that potent immune activation is induced by using a particle and an immuno-stimulating factor to be administered separately to each other, wherein the particle comprises an amphiphilic polymer in which a hydrophobic segment is poly(hydroxy acid) and a hydrophilic segment is polysaccharide, and a cancer antigen. The type of the immune response generated by the medicament of the present invention is not limited. Generally, it is known that there are Th1-type immune response and Th2-type immune response as the types of immune response, and that preferentially generated immune response among them depends on the types of an antigen, an administration site, an administration method, and an immuno-stimulating factor that is used with the particle in combination. The present invention may induce both Th1-type and Th2-type immune responses.

The antitumor activity of a combination of the particle comprising: an amphiphilic polymer in which a hydrophobic segment is poly(hydroxy acid) and a hydrophilic segment is polysaccharide, and a cancer antigen; with an immuno-stimulating factor, used in the present invention, may be evaluated by examining the inhibition of tumor growth in a cancer-bearing animal *in vivo,* as described below.

The amphiphilic polymer constituting a particle together with a cancer antigen, in the medicament of the present invention, is described. The term "amphiphilic" means having both properties of hydrophilicity and hydrophobicity. When the solubility in water of a certain portion (segment) is higher than that of other portions, the certain portion (segment) is referred to as being hydrophilic. It is desirable that the hydrophilic portion is soluble in water, but may be hardly soluble in water as long as it has higher solubility in water compared to other portions. When the solubility in water of a certain portion (segment) is lower than that of other portion, the certain portion (segment) is referred to as being hydrophobic. It is desirable that the hydrophobic portion is insoluble in water, but may be soluble in water as long as it has lower solubility in water as compared with other portions.

The term "amphiphilic polymer" means a polymer having the above amphiphilicity as the whole molecule. The amphiphilic "polymer" indicates an amphiphilic molecule having a hydrophilic segment or a hydrophobic segment, or both thereof with a molecular structure constituted from a repeated structure of a minimum unit (monomer). The structure of the amphiphilic polymer in the present invention is not particularly limited, and specific examples thereof include: a straight-chained block-type polymer in which polysaccharide and poly(hydroxy acid) are connected, a branched polymer having branches, comprising a plurality of polysaccharides or poly(hydroxy acids); a graft-type polymer comprising a main chain of polysaccharide and a side chain of poly(hydroxy acid); and a graft-type polymer comprising a main chain of poly(hydroxy acid) and a side chain of polysaccharide, and preferably, a straight-chained block-type polymer in which polysaccharide and poly(hydroxy acid) are connected.

In the present invention, the hydrophilic segment of the amphiphilic polymer is polysaccharide. The hydrophilic segment is not particularly limited as long as it is polysaccharide, and specific examples thereof include dextran, β-glucan, mannan, chitin, chitosan, gellan gum, alginic acid, hyaluronic acid or pullulan, and preferably dextran or β-glucan.

The glucan refers to glucose-containing polysaccharides, and the β-glucan refers to glucan containing one or more β-bonds between glucose subunits. In other words, the β-glucan for use in the present invention contains a β-bond, and may contain only a β-bond. The β-glucan for use in the present invention may be branched or linear. Examples of preferable β-glucan include β-glucan containing one or more β-1,3 bonds and/or one or more β-1,6 bonds, or β-glucan containing one or more β-1,2 bonds and/or a β-1,4 bond, and β-glucan containing one or more β-1,3 bonds and/or one or more β-1,6 bonds is more preferable, and β-glucan containing one or more β-1,3 bonds is further preferable. Specific examples of the β-glucan containing one or more β-1,3 bonds include curdlan, pachyman, laminaran, lichenan, schizophyllan, lentinan, scleroglucan, black yeast glucan (preferably, black yeast-derived β-1,3 glucan or β-1,6 glucan) or pachymaran, and preferably include curdlan, pachyman, laminaran, schizophyllan, scleroglucan, black yeast glucan or pachymaran.

Examples of linear β-glucan containing one or more β-1,3 bonds include β-glucan mainly containing a β-1,3 bond (for example, curdlan or pachyman), or β-glucan containing a β-1,3 bond and a β-bond other than a β-1,3 bond (for example, laminaran or lichenan).

Examples of branched β-glucan containing one or more β-1,3 bonds include β-glucan containing a β-1,3 bond and a β-1,6 bond (for example, schizophyllan or lentinan, scleroglucan, black yeast glucan).

The β-glucan for use in the present invention may be derivatized β-glucan. Examples of such derivatization include addition reaction of a carboxymethyl group, or oxidative cleavage reaction. Examples of the derivatized β-glucan include carboxymethyl curdlan obtained by adding a carboxymethyl group to curdlan, or pachymaran obtained by cleaving pachyman.

The number-average molecular weight of polysaccharide is not particularly limited, and is preferably 500 to 100,000, more preferably 500 to 50,000, further preferably 1,000 to 10,000, for example, 1,000 to 9,000, 1,000 to 8,000, 1,000 to 7,000, 1,000 to 6,000, 1,000 to 5,000, 1,000 to 4,000, 1,000 to 3,000. The number-average molecular weight is an average molecular weight calculated by a method in which weighting for the size of a molecule is not considered (simple average). The number-average molecular weight of polysaccharide may be determined by gel permeation chromatography (GPC).

The present invention is characterized in that the hydrophobic segment of the amphiphilic polymer is poly(hydroxy acid). The poly(hydroxy acid) is not particularly limited, and is preferably a biocompatible polymer without remarkable adverse effect upon administration into an organism. The biocompatibility herein refers to having an LD50 of 2,000 mg/kg or more when the polymer is orally administered to a rat. The poly(hydroxy acid) may be a copolymer of a plurality of types of hydroxy acids, and is preferably a polymer of two or less types of hydroxy acids.

Specific examples of the poly(hydroxy acid) preferably include polyglycolic acid, polylactic acid, poly(2-hydroxybutyric acid), poly(2-hydroxyvaleric acid), poly(2-hydroxycaproic acid), poly(2-hydroxycapric acid), poly(malic acid), or any derivative and copolymer of such polymer compounds, and preferably, poly(lactic-co-glycolic acid), polylactic acid or polyglycolic acid, and more preferably, poly(lactic-co-glycolic acid) . In a case where the poly(hydroxy acid) is poly(lactic-co-glycolic acid), the compositional ratio (lactic acid/glycolic acid) (mol/mol) of the poly(lactic-co-glycolic acid) is not particularly limited as long as the problems of the present invention are achieved, and is preferably 99/1 to 1/99, more preferably 80/20 to 20/80, for example, 60/40 to 40/60, or 50/50.

The number-average molecular weight of the poly(hydroxy acid) portion in the amphiphilic polymer in the present invention is not particularly limited, and is preferably 500 to 1,000,000, more preferably 500 to 100,000, further preferably 7,000 to 50,000, 7,000 to 50,000, 7,000 to 40,000, 7,000 to 30,000, 7,000 to 20,000, 7,000 to 15,000, 7,000 to 13,000, 7,000 to 12,000. The number-average molecular weight of poly(hydroxy acid) can be determined from the difference between the number-average molecular weight of the amphiphilic polymer, in which a hydrophobic segment is poly(hydroxy acid) and a hydrophilic segment is polysaccharide, and the number-average molecular weight of polysaccharide.

The number-average molecular weight of the amphiphilic polymer constituting the particle is not particularly limited, and is preferably 1,000 to 1,000,000, more preferably 1,000 to 100,000, further preferably 9,000 to 50,000, 9,000 to 50,000, 9,000 to 40,000, 9,000 to 30,000, 9,000 to 20,000, 9,000 to 15,000. The number-average molecular weight of the amphiphilic polymer can be determined by gel permeation chromatography (GPC).

The amphiphilic polymer may be produced by a known method, and specific examples of the method include a production method comprising adding poly(hydroxy acid) to polysaccharide and performing a condensation reaction, or a production method comprising adding a hydroxy acid activated monomer to polysaccharide and performing a polymerization reaction.

In a case where the amphiphilic polymer is a straight-chained block-type polymer in which polysaccharide and poly(hydroxy acid) are connected, the polymer may be produced by a known method, and specific examples of the method include a production method comprising performing a condensation reaction of a poly(hydroxy acid) copolymer at a reducing terminal of polysaccharide of the amphiphilic polymer using an activator of a terminal functional group [Macromol. Rapid Commun., 31, p.1664-1684 (2010)].

In a case where the amphiphilic polymer is a graft-type polymer comprising a main chain of polysaccharide and a side chain of poly(hydroxy acid), the polymer may be produced according to the following method (1), (2), or (3).

(1) A method of producing a graft-type polymer, in which a poly(hydroxy acid) is introduced by adding a hydroxy acid activated monomer to polysaccharide to perform polymerization reaction in the presence of a tin catalyst [Macromolecules, 31, p. 1032-1039 (1998)]
(2) A method of producing a graft-type polymer, in which a graft chain of poly(hydroxy acid) is introduced by activating some unprotected hydroxyl groups of polysaccharides in which most hydroxyl groups are protected with substituents, with a base, then adding a hydroxy acid activated monomer thereto, and protect groups are finally removed [Polymer, 44, p. 3927-3933, (2003)]
(3) A method of producing a graft-type polymer, in which the condensation reaction of a copolymer of poly(hydroxy acid) to polysaccharide is performed by using a dehydrating agent and/or an activator of a functional group [Macromolecules, 33, p. 3680-3685 (2000)].

The amphiphilic polymer is preferably water-insoluble as a whole in order to keep immuno-stimulating action for a long period and not to be rapidly excreted *in vivo.* The term "water-insoluble" herein refers to having a solubility in water of 1 g (amphiphilic polymer)/100 ml (water) or less.

The amphiphilic polymer may be a modified amphiphilic polymer to which a desired function is imparted by modification with a functional compound. As a specific example, the amphiphilic polymer may be a modified amphiphilic polymer in which an immuno-stimulating factor described below is bound to the amphiphilic polymer by a known method, in order to enhance immune activation action.

The cancer antigen constituting the particle together with the amphiphilic polymer is described. The "cancer antigen" in the present invention is a substance which triggers immune response *in vivo* and thus can be utilized as a medicine or vaccine for treatment and/or prevention of cancer. The particle comprising the amphiphilic polymer and the cancer antigen, of the present invention, can be adopted as an active ingredient to enhance an immune response triggered by the cancer antigen.

The cancer antigen comprised in the particle of the present invention is not particularly limited as long as the objects of the present invention are achieved. Examples of the cancer antigen include peptide, protein, glycoprotein, glycolipid, lipid, carbohydrate, nucleic acid, and polysaccharide, as well as any virus, fungus body, tissue and cell comprising any of them. Specific examples include, cancer antigens described in WO2017/181128, such as WT1, MUC1, LMP2, HPV E6, HPV E7, EGFRvIII, Her-2/neu, idiotype, MAGE A3, p53, NY-ESO-1 (CTAG 1), PSMA, CEA, MelanA/Mart1, Ras, gp100, proteinase 3, bcr-able, tyrosinase, survivin, PSA, hTERT, sarcoma translocation breakpoints, EphA2, PAP, MP-IAP, AFP, EpCAM, ERG, NA17-A, PAX3, ALK, androgen receptor, cyclin B1, MYCN, PhoC, TRP-2, mesothelin, PSCA, MAGE A1, CYP1B1, PLAC1, BORIS, ETV6-AML, NY-BR-1, RGS5, SART3, carbonic anhydrase IX, PAX5, OY-TES1, sperm protein 17, LCK, HMWMAA, AKAP-4, SSX2, XAGE1, B7-H3, legumain, Tie2, Page4, VEGFR2, MAD-CT-1, FAP, PAP, PDGFR-beta, MAD-CT-2, CEA, TRP-1(gp75), BAGE1, BAGE2, BAGE3, BAGE4, BAGE5, CAMEL, MAGE-A2, MAGE-A4, MAGE-A5, MAGE-A6, MAGE-A8, MAGE-A9, MAGE-A10, MAGE-A11, MAGE-A12 and Fos-associated antigen 1. As a model experiment system to evaluate an enhancement in antitumor effect of a cancer antigen by a particulate adjuvant, an experiment system which allows a particulate model cancer antigen, such as ovalbumin (OVA), to act on cancer cells in which the model cancer antigen is forcedly expressed, and the cancer antigen herein also encompasses such a model cancer antigen. The number of cancer antigens comprised in the particle of the present invention is not particularly limited, and may only one kind, or two or more kinds.

An additional component other than the cancer antigen and the amphiphilic polymer may be comprised in the particle which is one active ingredient in the present invention and which is composed of the amphiphilic polymer and the antigen. Specific examples of the additional component include well-known lipid as a component of a liposome or lipid nanoparticle, a particle capable of enclosing the antigen, such as a cancer antigen. However, as clear from Examples, the expected effects are sufficiently obtained in the present invention even without using any lipid in a component other than the antigen constituting the particle, and thus no lipid is preferably comprised as the additional component constituting the particle.

The structure of the particle, which is one active ingredient in the present invention and is composed of the amphiphilic polymer and the cancer antigen, is not particularly limited. Since the particle is a complex of the amphiphilic polymer and the cancer antigen, a structure in which the hydrophilic segment of the amphiphilic polymer constituting the particle is located inside the particle and the hydrophobic segment serves as an outer layer of the particle is preferable because it is formed the cancer antigen is enclosed inside the particle and may be more stably maintained.

The method for producing the particle is not particularly limited, and examples thereof include a drying in liquid method, a spray-drying method, and a pulverizing method, and the particle in the present invention is preferably produced by a drying in liquid method.

Examples of the method for producing the particle by a drying method in liquid include an O/W emulsion method, a W/O/W emulsion method, and a S/O/W emulsion method.

The particle, when produced by an O/W emulsion method, may be produced by, for example, a step of preparing an O/W emulsion solution by mixing a water-immiscible organic solvent dissolving a powder of the amphiphilic polymer constituting the particle, and an aqueous solution dissolving a surface modifier and a cancer antigen, and a step of obtaining the particle by removing the water-immiscible organic solvent from the O/W emulsion solution.

The particle, when produced by a W/O/W emulsion method, may be produced by, for example, a step of preparing a W/O emulsion solution by mixing an aqueous solvent dissolving a cancer antigen and a water-immiscible organic solvent dissolving a powder of the amphiphilic polymer constituting the particle, a step of preparing a W/O/W emulsion solution by mixing the W/O emulsion solution and an aqueous surface modifier solution, and a step of obtaining the particle by removing the water-immiscible organic solvent from the W/O/W emulsion solution.

The particle, when produced by a S/O/W emulsion method, may be produced by, for example, a step of preparing a W/O emulsion solution by mixing an aqueous solvent dissolving a cancer antigen and a water-immiscible organic solvent dissolving a powder of the amphiphilic polymer constituting the particle, a step of obtaining a solid content by removing the solvent from the W/O emulsion solution, a step of obtaining a S/O suspension solution by dispersing the solid content in the water-immiscible organic solvent, a step of preparing a S/O/W emulsion solution by mixing the S/O suspension solution and an aqueous surface modifier solution, and a step of obtaining the particle by removing the water-immiscible organic solvent from the S/O/W emulsion solution.

The content ratio of the antigen in the particle (antigen/particle) is preferably 0.01 to 20% by weight, more preferably 0.1 to 10% by weight. Examples of the method for quantitatively determining the content ratio of the antigen include a method comprising extracting the antigen from the particle using an organic solvent and quantifying the content ratio of the antigen by gel electrophoresis or liquid chromatography.

The surface modifier used for particle preparation is preferably a water-soluble polymer or a surfactant. The water-soluble polymer herein is a polymer compound having a solubility in water of 1 g (water-soluble polymer)/100 ml (water) or more.

Examples of the water-soluble polymer serving as the surface modifier include polyethylene glycol, polyvinylpyrrolidone, polyvinyl alcohol, polyethylenimine, polyacrylic acid, polymethacrylic acid, poly-1,3-dioxolane, 2-methacryloyloxyethyl phosphorylcholine polymer, poly-1,3,6-trioxane, polyamino acid, peptide, protein, sugar (monosaccharides, oligosaccharides, and polysaccharides), and preferably polyvinyl alcohol.

Examples of the surfactant serving as the surface modifier include a nonion activator such as polyoxyethylene polyoxypropylene glycol, sucrose fatty acid ester, polyoxyethylene fatty acid ester, polyoxyethylene sorbitan mono-fatty acid ester, polyoxyethylene sorbitan di-fatty acid ester, polyoxyethylene glycerin mono-fatty acid ester, polyoxyethylene glycerin di-fatty acid ester, polyglyceryl fatty acid ester, polyoxyethylene castor oil, or polyoxyethylene hydrogenated castor oil, alkyl sulfate such as sodium lauryl sulfate, ammonium lauryl sulfate, or sodium stearyl sulfate, or lecithin, and preferably polyoxyethylene polyoxypropylene glycol.

The water-immiscible organic solvent used in particle preparation preferably can dissolve the amphiphilic polymer and hardly dissolves or does not dissolve polysaccharide. The solubility in water of the water-immiscible organic solvent is preferably 30 g (water-immiscible organic solvent)/100 ml (water) or less. Specific examples of the water-immiscible organic solvent include ethyl acetate, isopropyl acetate, butyl acetate, dimethyl carbonate, diethyl carbonate, methylene chloride, and chloroform.

The aqueous solvent used in particle preparation is an aqueous solution containing water and optionally a water-soluble component. Examples of the water-soluble component include inorganic salts, sugars, organic bases, amino acids, peptides, proteins, and nucleic acids.

The surface modifier used in the above production process may be bound to the particle surface. The bond herein may be a non-covalent bond or a covalent bond. The non-covalent bond is preferably hydrophobic interaction, and may be ion bond (electrostatic interaction), hydrogen bond, coordinate bond, van der Waals binding, or physical adsorption, or may be a combination thereof.

The average particle size of the particle is preferably 0.1 µm to 50 µm, more preferably 0.1 µm to 25 µm, further preferably 0.1 µm to 10 µm, particularly preferably 0.1 µm to 1 µm, for example, 0.2 µm to 0.9 µm, 0.3 µm to 0.8 µm, or 0.4 µm to 0.7 µm. The average particle size herein may be determined with a dynamic light scattering apparatus (DLS: for example, ELS-Z, OTSUKA ELECTRONICS CO., LTD) according to a cumulant method.

The term "treatment" used herein means treatment of cancer based on the antitumor effect described above. The term "prevention" used herein means not only prevention of development of cancer, but also prevention of metastasis or recurrence of cancer.

The terms "tumor" and "cancer" used herein means malignant neoplasm, and are interchangeably used.

The method for administering the particle of the medicament of the present invention to an organism (subject) is not particularly limited, and examples thereof include subcutaneous administration, intracutaneous administration, intramuscular administration, intranasal administration, transpulmonary administration, oral administration, percutaneous administration, sublingual administration, intravaginal administration, intraperitoneal administration, and lymph node administration, and preferably, intracutaneous administration or subcutaneous administration.

Preferably, an administration method different from the administration of an immuno-stimulating factor described below may be selected as the administration method of the particle. For example, when administration of an immuno-stimulating factor is performed by administration to a local tumor area, administration of the particle may be performed by subcutaneous administration.

The dose of the particle is appropriately set depending on the administration method and the frequency of administration, and, for example, when the particle of the present invention is subcutaneously administered to humans, a single dose may be selected within a range of 0.0001 mg to 1000 mg per kg of body weight. Alternatively, the dose may be selected within a range of, for example, 0.001 mg/body to 100000 mg/body per patient, or for example, 1 mg to 30 mg per kg of patient's body weight, but is not necessarily limited thereto. The dose and the administration method are varied depending on weight, age, gender, and symptoms of the patient, and may be appropriately selected by persons skilled in the art.

The immuno-stimulating factor as one active ingredient in the present invention is described. The term "immuno-stimulating factor" herein means a substance which is a factor activating one or more immune cells and maintaining and/or enhancing the immune function of such cells. The term "immune cells" herein include, for example, T lymphocytes, B lymphocytes, NK cells, monocytes, dendritic cells, granulocytes, macrophages, bone marrow-derived inhibitory cells, Langerhance cells and a group of precursor cells thereof, and a group of the immune cells in tumors.

The immuno-stimulating factor for use in the present invention is not particularly limited, and specific examples thereof include a ligand or agonist binding to a Toll-like receptor (TLR), a NOD-like receptor (NLR), a RIG-like receptor, a C-type lectin receptor (CLR) or a stimulator of interferon gene (STING), and preferably a ligand or agonist binding to TLR.

Specific examples of TLR include TLR2, TLR3, TLR4, TLR5, TLR7, TLR8, TLR9 or TLR11, and specific examples of the ligand or agonist binding thereto include the following (i) to (vii):
(i) a ligand or agonist binding to TLR2 selected from the group consisting of peptidoglycan, lipoprotein, lipopolysaccharide, and zymosan.
(ii) a ligand or agonist binding to TLR3 selected from the group consisting of poly(I:C) and poly(A:U).
(iii) a ligand or agonist binding to TLR4 selected from the group consisting of lipopolysaccharide (LPS), HSP60, RS09, and MPLA.
(iv) flagellin as a ligand or agonist binding to TLR5.
(v) a ligand or agonist binding to TLR7 or 8 selected from the group consisting of an imidazoquinoline compound and single-strand RNA.
(vi) a ligand or agonist binding to TLR9 selected from the group consisting of bacterial DNA, unmethylated CpG DNA, hemozorin, ODN1585, ODN1668 and ODN1826.
(vii) a ligand or agonist binding to TLR11 selected from the group consisting of profilin and uropathogenic bacteria.

The immuno-stimulating factor in the present invention is preferably a ligand or agonist binding to TLR3, or a ligand or agonist binding to TLR7 or/and 8, more preferably the ligand or agonist of the above item (ii) or (v).

Regarding the above item (ii) above, which is a ligand or agonist binding to TLR3 selected from the group consisting of poly(I:C) and poly(A:U), poly(I:C) is preferred. Poly(I:C) is double-stranded RNA formed by an RNA chain only comprising inosine as a base and an RNA chain only comprising cytidine as a base. The chain length of poly(I:C) used in the present invention is not particularly limited.

The imidazoquinoline compound in above item (v) is not particularly limited as long as it can bind to TLR7 or TLR8, preferable specific examples thereof include any compound described in US. Patent No. 8,951,528 and any compound described in WO2015/103989, and examples thereof include 4-amino-2-(ethoxymethyl)-a,a-dimethyl-1H-imidazo[4,5-c]quinoline-1-ethanol (Resiquimod (R848)), 1-(2-methylpropyl)-1H-imidazo[4,5-c]quinoline-4-amine (Imiquimod), 1-(4-amino-2-ethylaminomethylimidazo-[4,5-c]quinolin-1-yl)-2-methylpropan-2-ol (Gardiquimod), N-[4-(4-amino-2-ethyl-1H-imidazo[4,5-c]quinolin-1-yl)butyl-]methanesulfonamide (PF-4878691), 4-amino-aa-dimethyl-2-methoxyethyl-1H-imidazo[4,5-c]quinoline-1-ethanol, 1-(2-(3-(benzyloxy)propoxy)ethyl)-2-(ethoxymethyl)-1H-imidazo[4,5-c]quinolin-4-amine, 4-amino-2-ethoxymethyl-aa-dimethyl-6,7,8,9-tetrahydro-1H-imidazo[4,5-c]quinoline-1-ethanol, N-(2-{2-[4-amino-2-(2-methoxyethyl)-1H-imidazo[4,5-c]quinolin-1-yl]eyhoxy} ethyl)-n'-phenylurea, 1-2-amino-2-methylpropyl)-2-(ethoxymethyl)-1H-imidazo[4,5-c]quinolin-4-amine, 1-{4-[(3,5-dichlorophenyl)sulfonyl]butyl}-2-ethyl-1H-imidazo[4,5-c]quinolin-4-amine, N-(2-{2-[4-amino-2-(ethoxymethyl)-1H-imidazo[4,5-c] quinolin -1-yl] ethoxy } ethyl)- n' -cyclohexylurea, N-{3-[4-amino-2-(ethoxymethyl)-1H-imidazo[4,5-c]quinolin-1-yl]propyl}-n'-(3-cyanophenyl)thiourea, N-[3-(4-amino-2-butyl-1H-imidazo[4,5-c]quinolin-1-yl)-2,2-dimethylpropyl]benzamide, 2-butyl-1-[3-(methylsulfonyl)propyl]-1H-imidazo[4,5-c]quinolin-4-amine, and derivatives thereof. More preferred is imiquimod, and imiquimod is a compound with a molecular weight of approximately 240.3, which is represented by CAS No. 99011-02-6. The IUPAC name of imiquimod is 4-amino-1-(2-methylpropyl)-1H-imidazo[4,5-c]quinoline, and another nomenclature is R837, S-26308, 1-(2-METHYLPROPYL)-1H-IMIDAZO[4,5-C]QUINOLIN-4-AMINE; 4-amino-1-isobutyl-1h-imidazo[4,5-c]quinoline, 1-isoButyl-1H-Inudazole[4.5-c]quinoline-4-amine; 1-(2-Methylpropyl)-1H-imidazo[4,5-c]quinolin-4-amine.

Specific examples of the ligand or agonist binding to a NOD-like receptor (NLR) include M-TriDAP and PGN. In addition, examples of the ligand or agonist binding to NOD1 also include Tri-DAP, iE-DAP, and C12-iE. In addition, examples of the ligand or agonist binding to NOD2 include MDP, N-glycosyl-MDP, Murabutide, M-TriLyS-D-ASN, M-TriLYS, and L18-MDP.

Specific examples of the ligand or agonist binding to a RIG-like receptor include 5'ppp-dsRNA, poly(dA:dT), poly(dG:dC), and poly(I:C).

Specific examples of the ligand or agonist binding to a C-type lectin receptor (CLR) include trehalose 6,6-dibehenate, zymosan, WGP, HKSC, HKCA, and curdlan AL.

Specific examples of the ligand or agonist binding to a stimulator of interferon gene (STING) include c-di-GMP, c-di-AMP, 2'3'-cGAMP, 3'3'-cGAMP, or 2'2'-cGAMP.

Imiquimod to be used may be obtained by chemical synthesis according to a technique known to persons skilled in the art and may also be a commercially available medicament. The dosage form of imiquimod for administration to patients is preferably a form for the percutaneous administration, preferably a cream formulation. As medicaments comprising imiquimod as active ingredients, "beselna cream 5%" in Japan and "Aldara (registered trademark) Cream, 5%" in Europe and the United States are on the market, and such a medicament may be administered according to the method and dose described in the instruction when imiquimod is used in the present invention.

The term "use in combination" or "combination" herein refers to simultaneous administration or administration of each of the particle and the immuno-stimulating factor as independent formulations (namely, separately) in a predetermined interval to the same organism. The interval between one administration and another administration is not particularly limited. The interval may be simultaneous administration to one administration, or may be 30 minutes later, 1 hour later, 3 hours later, 6 hours later, 12 hours later, 1 day later, 3 days later, 5 days later, 7 days later, 2 weeks later, 3 weeks later, or 4 weeks later from one administration. At the time when the particle or the immuno-stimulating factor may exhibit its activity *in vivo,* at least any one thereof is preferably administered. In addition, at the time before losing the activity of one of the particle or the immuno-stimulating factor, which is administered first, *in vivo,* the other is preferably administered. The particle may be administered first, or the immuno-stimulating factor may be administered first.

The "combination medicament" herein refers to a medicament comprising a plurality of formulations for use in combination described above independent from each other. The combination medicament of the present invention is a medicament used for treatment and/or prevention, and comprises the particle and the immuno-stimulating factor as respective independent formulations. Use of the combination medicament of the present invention is preferably characterized in that administration of the particle and administration of the immuno-stimulating factor are separately performed by administration methods different from each other. The treatment and/or prevention in which the combination medicament of the present invention is used are/is according to a method for treating and/or preventing cancer described below.

The method for administering the immuno-stimulating factor is not particularly limited as long as the objects of the present invention are achieved, and is preferably an administration route different from the administration route of the particle, preferably administration to a local tumor area. The administration route may be an oral or parenteral route, and specific examples of a dosage form include the form of an injectable agent, the form of an agent for intranasal administration, the form of an agent for transpulmonary administration, and the form for percutaneous administration. In the case of the form of an injectable agent, administration may be systemically or locally, and may be, for example, intravenous injection, intramuscular injection, intraperitoneal injection, subcutaneous injection, or intratumoral injection, and is preferably administered by intratumoral injection. Such a form for percutaneous administration is, for example, an agent called liniment or external medicine. Examples of external medicine include a solid agent, a solution, spray, ointment, cream, and gel, and preferably cream is percutaneously administered.

The administration method used for administration to a local tumor area is not particularly limited as long as an active ingredient can reach a target tumor without systemic circulation. For example, the method may be any method that may retain an active ingredient in a tumor or around a tumor. Specifically, for example, administration with an injection such as an intratumoral injection or a subcutaneous injection to the skin surrounding a tumor, or percutaneous administration such as application of an external medicine to the skin surface surrounding a tumor may be used as local administration to a tumor.

The medicament of the present invention may include, as an active ingredient, not only the particle and the immuno-stimulating factor, but also a known antitumor agent, such as in literature, as long as the effects of the medicament of the present invention are not impaired. Specific examples of known antitumor agent include, but are not particularly limited to, paclitaxel, doxorubicin, daunorubicin, cyclophosphamide, methotrexate, 5-fluorouracil, thiotepa, busulfan, improsulfan, piposulfan, benzodopa, carboquone, meturedopa, uredopa, altretamine, triethylenemelamine, triethylenephosphoramide, triethilenethiophosphoramide, trimethylolomelamine, bullatacin, bullatacinone, camptothecin, bryostatin, callystatin, cryptophycin 1, cryptophycin 8, dolastatin, duocarmycin, eleutherobin, pancratistatin, sarcodictyin, spongistatin, chlorambucil, chloRNAphazine, cholophosphamide, estramustine, ifosfamide, mechlorethamine, mechlorethamine oxide hydrochloride, melphalan, novembichin, phenesterine, prednimustine, trofosfamide, uracil mustard, carmustine, chlorozotocin, fotemustine, lomustine, nimustine, ranimustine, calicheamicin, dynemicin, clodronate, esperamicin, aclacinomycin, actinomycin, authramycin, azaserine, bleomycin, cactinomycin, carabicin, carminomycin, carzinophilin, chromomycin, dactinomycin, detorbicin, 6-diazo-5-oxo-L-norleucine, adriamycin, epirubicin, esorubicin, idarubicin, marcellomycin, mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin, denopterin, pteropterin, trimetrexate, fludarabine, 6-mercaptopurine, thiamiprine, thioguanine, ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone, aminoglutethimide, mitotane, trilostane, frolinic acid, aceglatone, aldophosphamide glycoside, aminolevulinic acid, eniluracil, amsacrine, bestrabucil, bisantrene, edatraxate, defofamine, demecolcine, diaziquone, elfornithine, elliptinium acetate, epothilone, etoglucid, lentinan, lonidamine, maytansine, ansamitocine, mitoguazone, mitoxantrone, mopidanmol, nitraerine, pentostatin, phenamet, pirarubicin, losoxantrone, podophyllinic acid, 2-ethylhydrazide, procarbazine, razoxane, rhizoxin, schizophyllan, spirogermanium, tenuazonic acid, triaziquone, roridine A, anguidine, urethane, vindesine, dacarbazine, mannomustine, mitobronitol, mitolactol, pipobroman, gacytosine, docetaxel, gemcitabine, 6-thioguanine, mercaptopurine, cisplatin, oxaliplatin, carboplatin, vinblastine, etoposide, mitoxantrone, vincristine, vinorelbine, novantrone, teniposide, edatrexate, daunomycin, aminopterin, xeloda, ibandronate, irinotecan, topoisomerase inhibitor, difluoromethylomithine (DMFO), retinoic acid, capecitabine, and pharmacologically acceptable (known) salts or (known) derivatives thereof.

The administration method of the antitumor agent used in combination is not particularly limited. For example, the method may be the same method as or a different method from the administration method of the particle or the immuno-stimulating factor. The administration method is an oral or parenteral method, and specific examples of a dosage form include the form of an injectable agent, the form of an agent for intranasal administration, the form of an agent for transpulmonary administration, and the form for percutaneous administration. In the case of the form of an injectable agent, administration may be systemically or locally, and may be, for example, intravenous injection, intramuscular injection, intraperitoneal injection, subcutaneous injection, or intratumoral injection. Such a form for percutaneous administration is, for example, an agent called liniment or external medicine. Examples of external medicine include a solid agent, a solution, spray, ointment, cream, and gel.

The treatment and/or prevention of cancer, according to the present invention, include(s) various modes other than administration of the medicament.

Specifically, the method for treating and/or preventing cancer of the present invention is not particularly limited as long as the objects of the present invention are achieved, preferably, and is characterized in that the particle (particle comprising an amphiphilic polymer in which a hydrophobic segment is poly(hydroxy acid) and a hydrophilic segment is polysaccharide, and a cancer antigen), and the immuno-stimulating factor are separately administered by administration methods different from each other. The method for treating and/or preventing cancer of the present invention preferably comprises a step of administering the particle to a subject and a step of administering the immuno-stimulating factor to the subject, and any of the steps may be first performed or both steps may be simultaneously performed as described with respect to the term "use in combination". A surgical procedure and/or administration of other medicament (for example, the above known antitumor agent) may also be additionally performed, if necessary.

For example, respective active ingredients in the combination medicament of the present invention may be administered simultaneously or individually in a staggered manner. As a specific example, these active ingredients may be administered within a time interval of up to approximately 3 weeks, namely, the second active ingredient may be administered from immediately after to up to approximately 3 weeks after the administration of the first active ingredient. Herein, for example, such administration may be performed subsequent to a surgical procedure, or a surgical procedure may be performed between the administrations of the first active ingredient and the second active ingredient. The medicament of the present invention may also be administered according to a plurality of administration cycles. For example, when each of the active ingredients of the medicament of the present invention is administered simultaneously, both medicaments comprising each active ingredient may be administered once per approximately 2 days to approximately 3 weeks as one cycle. This treatment cycle may be repeated afterwards, if necessary, according to the judgment of a physician in charge. Even when the first administrations of each active ingredient are simultaneously performed, subsequent administrations may be performed as administrations of medicaments comprising each ingredient in different administration cycles. In this case, with respect to administration other than the first administration, the administration period of each active ingredient is adjusted to affect the same period. Likewise, when scheduling a formulation in a staggered manner, each of the administration periods of each active ingredient is adjusted to affect the same period. The interval between cycles may vary from 0 to 2 months. The dose of each active ingredient for the therapeutic and/or preventive agent for cancer of the present invention may be set in the same way as the dose of each active ingredient for the medicament.

The administration period for each of the active ingredients is not particularly limited. Specifically, the administration period may be, for example, 3 days or more, 4 days or more, 5 days or more, 1 week or more, 10 days or more, or 2 weeks or more. The frequency of administration of each of the ingredients during the administration period is also not particularly limited. As described above, administration is preferably performed so that the activity of each of the active ingredients is obtained over the same administration period. Therefore, the number of administrations or the frequency of administration may be appropriately set depending on the period during which each of the active ingredients exhibits activity in the body. Specifically, for example, the number or the frequency may be set depending on the administration method, the doses, and the types of the active ingredients. For example, in the case of an injectable agent, the injectable agent may be administered at a frequency of one or more times per month, one or more times per 3 weeks, one or more times per 20 days, one or more times per 10 days, one or more times per week, or two or more times per week, without any particular limitations. For example, in the case of an external medicine, the external medicine may be administered at a frequency of one or more times per 10 days, one or more times per week, two or more times per week, one or more times per 3 days, one or more times per 2 days or one or more times per day.

The cancer as a target in the present invention is not particularly limited as long as the cancer antigen comprised in the particle is expressed. The cancer is preferably basal cell carcinoma, Paget's disease, skin cancer, breast cancer, kidney cancer, pancreatic cancer, colon cancer, lung cancer, brain tumor, gastric cancer, uterus cancer, ovarian cancer, prostate cancer, urinary bladder cancer, esophageal cancer, leukemia, lymphoma, liver cancer, gallbladder cancer, sarcoma, mastocytoma, adrenal cortex cancer, Ewing's tumor, Hodgkin's lymphoma, mesothelioma, multiple myeloma, testicle cancer, thyroid cancer, or head and neck cancer, as described above. More preferably, the cancer is palpable (touchable) cancer, subcutaneously existing cancer, intracutaneously existing cancer, superficial cancer, cancer existing in the dermis and cancer existing in a non-parenchymal organ. These cancers may be primary cancers, metastatic cancers, cancers that have metastasized, or cancers that have recurred.

More specific examples of the cancer include Bowen's disease, melanoma, prickle cell cancer, extramammary Paget's disease, mycosis fungoides, Sezary's syndrome, cutaneous T/NK-cell lymphoma, T-cell leukemia or lymphoma having a lesion only in the skin, cutaneous B-cell lymphoma (indolent group), cutaneous T-cell lymph mammary adenoma, complex mammary adenoma, malignant mixed tumor of mammary gland, intraductal papillary carcinoma of mammary gland, lung adenocarcinoma, squamous cell cancer, small cell cancer, large cell cancer, glioma which is a neuroepithelial tissue tumor, glioblastoma, neuroblastoma, ependymoma, neuronal tumor, neuroectodermal tumor, neurilemoma, neurofibromatosis, meningioma, chronic lymphocytic leukemia, lymphoma, alimentary lymphoma, gastrointestinal lymphoma, small to medium cell lymphoma, cecal cancer, ascending colon cancer, descending colon cancer, transverse colon cancer, sigmoid colon cancer, rectal cancer, ovarian epithelial cancer, germ cell tumor, interstitial cell tumor, pancreatic duct cancer, invasive pancreatic duct cancer, adenocarcinoma of pancreatic cancer, acinar cell cancer, adenosquamous cancer, giant cell tumor, intraductal papillary mucinous tumor, mucinous cystadenocarcinoma, pancreatoblastoma, pancreatic islet cell tumor, Frantz's tumor, serous cystadenocarcinoma, solid pseudopapillary cancer, gastrinoma, glucagonoma, insulinoma, multiple endocrine neoplasia type-1 (Wermer syndrome), nonfunctional islet cell tumor, somatostatinoma, VIPoma, uterine cervical cancer, uterine body cancer, fibrosarcoma, osteosarcoma, joint sarcoma, Ewing sarcoma, Wilms's tumor, hepatoblastoma, soft tissue sarcoma, acute leukemia, chronic leukemia, spinal cord tumor, malignant soft tissue tumor, tumors of teratoma group, and head and neck cancer including hypopharynx cancer, oropharynx cancer, tongue cancer, nasopharyngeal cancer, oral cavity cancer, lip cancer, sinus cancer, and laryngeal cancer, but are not limited thereto. The cancer also includes palpable cancer, subcutaneously existing cancer, intracutaneously existing cancer, superficial cancer, cancer existing in the dermis, and cancer existing in a non-parenchymal organ, which each originate from the cancer described above. The cancer also includes palpable cancer, subcutaneously existing cancer, intracutaneously existing cancer, superficial cancer, cancer existing in the dermis and cancer existing in a non-parenchymal organ, which each originate from the cancer described above and have metastasized and recurred.

The combination of the particle and the immuno-stimulating factor, as the medicament of the present invention, has cytotoxic activity *in vivo.* Accordingly, the antitumor effect of the present invention may be determined by examining cytotoxic activity against cancer. The cytotoxic activity may be evaluated by administering the particle and the immuno-stimulating factor in combination, to an organism (subject) having cancer, measuring the size of a tumor after administration, and examining the size of the cancer over time. The antitumor effect of the present invention may also be evaluated by examining the survival rate of the subject. The antitumor effect may also be evaluated by examining the ability to produce cytokine or chemokine. The antitumor effect of the combination of the particle and the immuno-stimulating factor according to the present invention may also be determined by examining prevention of cancer, prevention of metastasis, or prevention of recurrence.

A preferred subject (patient) to be targeted is a mammal, and for examples, mammals include primates, pet animals, livestock animals, and sport animals, particularly preferably, humans, dogs and cats.

The medicament of the present invention may be formulated by a method known to persons skilled in the art. For example, the medicament can be parenterally used in an injectable agent such as an aseptic solution or a suspension liquid mixed with water or a pharmacologically acceptable liquid other than water. For example, the medicament may be formulated by appropriate combination with a pharmacologically acceptable carrier or medium, specifically, sterilized water, physiological saline, plant oil, an emulsifier, a suspension, a surfactant, a stabilizer, a fragrance, an excipient, or a binder, and mixing of the resultant in a unit dosage form required for a generally acceptable pharmaceutical formulation. The amount of an active ingredient in such a formulation is determined so that an appropriate dosage within a specified range is obtained.

An aseptic composition for injection may be prepared according to general formulation practice by using a vehicle such as distilled water for injection. Examples of an aqueous solution for injection include physiological saline, and an isotonic solution comprising glucose and other supporting agents, such as an aqueous solution including D-sorbitol, D-mannose, D-mannitol, or sodium chloride, and an appropriate dissolution aid, for example, alcohol, specifically, ethanol, polyalcohol such as propylene glycol or polyethylene glycol, and/or a nonion surfactant such as polysorbate 80(TM) or HCO-60 may be used in combination. An oily liquid is, for example, sesame oil or soybean oil, and a dissolution aid such as benzyl benzoate or benzyl alcohol may be used in combination. Such an oily liquid may also be mixed with a buffering agent such as a phosphate buffer solution or a sodium acetate buffer solution, a soothing agent such as procaine hydrochloride, a stabilizer such as benzyl alcohol or phenol, or an antioxidant. In general, a formulated injection solution is loaded into an appropriate ampoule.

Further aspects are described below.

The present invention also relates to the use of each active ingredient combined with the other active ingredient, as described above. Specifically, the invention relates to the use of the particle (particle comprising an amphiphilic polymer in which a hydrophobic segment is poly(hydroxy acid) and a hydrophilic segment is polysaccharide, and a cancer antigen) for treatment and/or prevention of cancer. The use herein comprises administering the particle to a subject in combination with an immuno-stimulating factor, and preferably, administration of the particle and administration of the immuno-stimulating factor are separately performed by different administration methods.

Similarly, the present invention also relates to the use of an immuno-stimulating factor for treatment and/or prevention of cancer. The use herein comprises administering the immuno-stimulating factor to a subject in combination with the particle, and preferably, administration of the particle and administration of the immuno-stimulating factor are separately performed by different administration methods.

The present invention further relates to a composition comprising each active ingredient for use in the method described above. Specifically, the invention relates to a composition comprising the particle, for use in the treatment and/or prevention of cancer. The use herein comprises administering the composition to a subject in combination with an immuno-stimulating factor, and preferably, administration of the composition and administration of the immuno-stimulating factor are separately performed by different administration methods. The use may comprise, for example, a step of administering the composition to a subject and a step of administering the immuno-stimulating factor to a subject.

Similarly, the present invention relates to a composition comprising an immuno-stimulating factor, for use in the treatment and/or prevention of cancer. The use herein comprises administering the composition to a subject in combination with the particle, and preferably, administration of the particle and administration of the composition are separately performed by different administration methods. The use may comprise, for example, a step of administering the particle to a subject and a step of administering the composition to a subject. Herein, such administration of the composition may be performed with the same dose, administration method, and frequency of administration as described above with respect to the administration of the active ingredient.

The use in each of these aspects is according to the above content described with respect to the method for treating and/or preventing cancer and the use of the medicament of the present invention. For example, such each use may comprise a step of administering the particle to a subject and a step of administering the immuno-stimulating factor to a subject, and for the administration of each active ingredient, dose, administration method, administration interval, administration period, frequency of administration, and the like described above with respect to each active ingredient may be used.

### Examples

Examples are shown below, but the present invention is not limited by these Examples.

### (Example 1) Synthesis of dextran-PLGA

In the present Example, poly(lactic-co-glycolic acid) (PLGA) was added to dextran to perform condensation reaction, thereby synthesizing a straight-chained block-type polymer.

### <Synthesis of dextran with a primary amino group at reducing terminal (dextran-NH₂)>

Sodium triacetoxyborohydride (203.5 mg) and N-Boc-ethylenediamine (76.1 µl) were loaded into a dimethylsulfoxide solution of dextran (number-average molecular weight 2,100, Pharmacosmos A/S)with a concentration of 500 mg/2 ml, and then reacted with stirring at 60°C for 91 hours. Next, unreacted N-Boc-ethylenediamine was removed by dialysis with water as an external solution, followed by freeze-drying to synthesize dextran-NHBoc. Next, deprotection reaction of a Boc terminal group of such dextran-NHBoc (450 mg) (aqueous 35% hydrochloric acid solution (5 ml)/dimethylsulfoxide (5 ml), stirred at ambient temperature for 35 hours) was performed, and then purification by water dialysis was performed, and a polymer powder (dextran-NH₂) was obtained by freeze-drying.

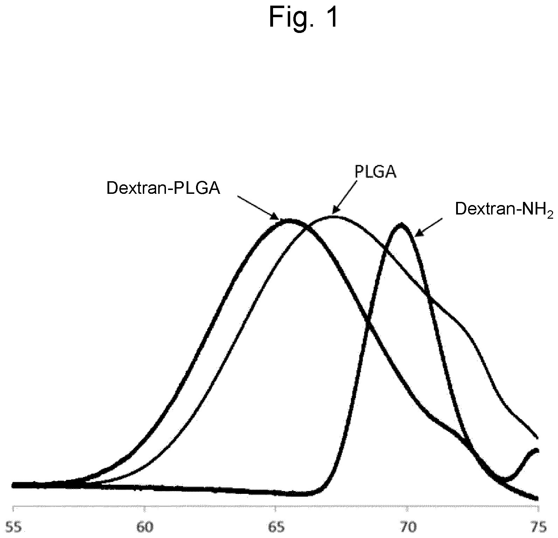

The number-average molecular weight of the dextran-NH₂ was determined by GPC measurement (column: TSK-gel α-5000 × 2, manufactured by Tosoh Corporation, DMF-based solvent, detector: RI, standard product: pullulan) (Figure 1: dextran-NH₂). It was confirmed by ¹H-NMR measurement that a primary amino group was introduced to a reducing terminal of dextran.

### <Synthesis of dextran-PLGA>

Poly(lactic-co-glycolic acid) having a heterobifunctional terminal group (COOH-PLGA-OH, FUJIFILM Wako Pure Chemical Corporation, PLGA-5020, number-average molecular weight 8,900) (350 mg) was mixed in a dimethylsulfoxide (0.58 ml) solution of 1-ethyl-3-(3-dimethylaminopropyl)carbodiimide hydrochloride (EDC) (35.5 mg) and N-hydroxysuccinimide (NHS) (21.3 mg), and then reacted at 50°C for 2 hours, thereby converting a carboxyl group at the α-terminal into an active ester (NHS). The reaction solution was loaded with dextran to which a primary amino group was introduced into a reducing terminal (dextran-NH₂, number-average molecular weight 2,700) (100 mg), and condensation reaction of an active ester (NHS) of NHS-PLGA-OH and the primary amino group of dextran-NH₂ was performed for 110.5 hours. After the reaction, unreacted dextran-NH₂ was removed by water dialysis, and unreacted NHS-PLGA-OH was removed by ultra-centrifugal purification to obtain a dextran-PLGA polymer 1.

A dextran-PLGA polymer 2 was obtained by the same method as for the dextran-PLGA polymer 1, using dextran-NH₂ (number-average molecular weight 1,800) and PLGA-5020 (number-average molecular weight 8,900).

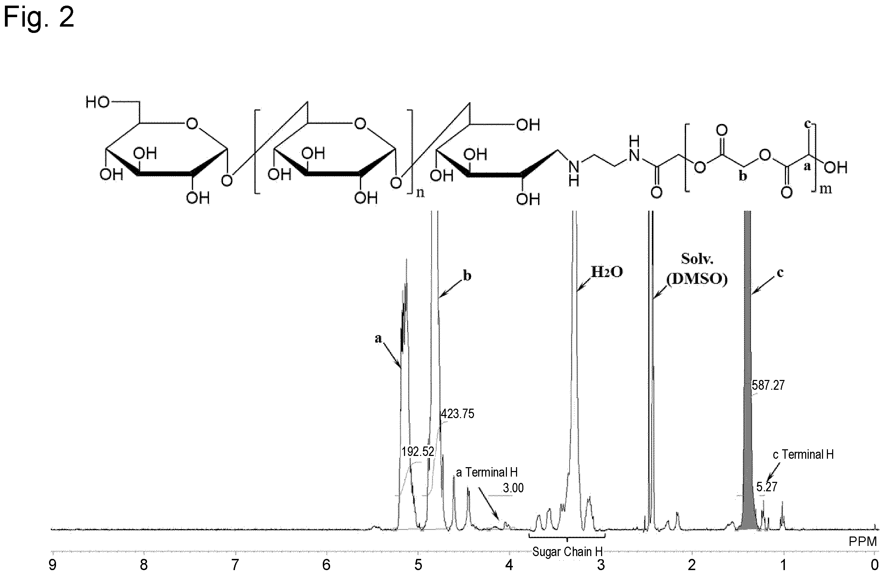

The number-average molecular weight of dextran-PLGA was determined by GPC measurement (column: TSK-gel α-5000 × 2, Tosoh Corporation, DMF-based solvent, detector: RI, standard product: pullulan) (Table 1, Figure 1: dextran-PLGA). It was confirmed by ¹H-NMR measurement that condensation reaction progressed (Figure 2: dextran-PLGA).

**[Table 1]**

| Results of analysis on dextran-PLGA (1, 2) | | | | |
|---|---|---|---|---|
| Amphiphilic polymer | Number-average molecular weight | Hydrophilic polysaccharide portion | Number-average molecular weight of hydrophilic polysaccharide | Number-average molecular weight of PLGA |
| 1 | 14500 | Dextran | 2700 | 11800 |
| 2 | 13400 | Dextran | 1800 | 11600 |

### (Example 2) Synthesis of black yeast glucan-PLGA

In the present Example, PLGA was added to black yeast glucan to perform condensation reaction, thereby synthesizing a straight-chained block-type polymer.

### <Synthesis of black yeast glucan with a primary amino group at reducing terminal (black yeast glucan-NH₂)>

After 5 g of black yeast glucan (DAISO CO.,LTD.) was dissolved in 150 ml of dimethylsulfoxide, 5 ml of an aqueous 35% hydrochloric acid solution was added, and the resultant was stirred at 105°C for 20 minutes. The reaction solution was transferred to a dialysis membrane, and dialysis was performed in water, followed by freeze-drying to obtain a black yeast glucan hydrolysate (number-average molecular weight 2,400) as a powder.

Sodium triacetoxyborohydride (413.3 mg) and N-Boc-ethylenediamine (246.9 µL) were loaded into a dimethylsulfoxide solution of a black yeast glucan hydrolysate (number-average molecular weight 2,400) having a concentration of 2.5 g/8 ml, and then reacted with stirring at 55°C for 169.5 hours. Next, unreacted N-Boc-ethylenediamine was removed by dialysis with water as an external solution, and black yeast glucan-NHBoc was obtained. After deprotection reaction of a Boc terminal group of the black yeast glucan-NHBoc (1.19 g) (aqueous 35% hydrochloric acid solution (14.3 ml)/dimethylsulfoxide (24 ml), stirred at ambient temperature for 2 hours), purification by water dialysis was performed, and a polymer powder (black yeast glucan-NH₂) was obtained by freeze-drying.

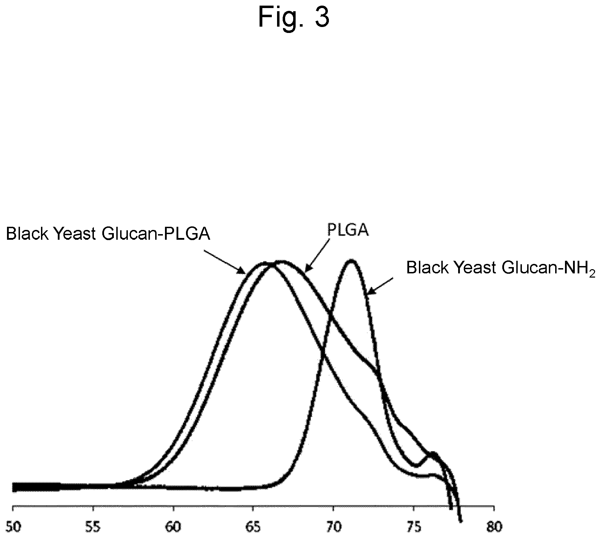

The number-average molecular weight of the black yeast glucan-NH₂ was determined by GPC measurement (column: TSK-gel α-5000 × 2, manufactured by Tosoh Corporation, DMF-based solvent, detector: RI, standard product: pullulan) (Figure 3: black yeast glucan-NH₂). It was confirmed by ¹H-NMR measurement that a primary amino group was introduced into a reducing terminal of black yeast glucan.

### <Synthesis of black yeast glucan-PLGA>

Poly(lactic-co-glycolic acid) having a heterobifunctional terminal group (COOH-PLGA-OH, FUJIFILM Wako Pure Chemical Corporation, PLGA-5020, number-average molecular weight 8,900) (4.462 g) was mixed in a DMSO (2.5 ml) solution of EDC (479.25 mg) and NHS (287.73 mg), and then reacted at 50°C for approximately 18 hours, thereby converting a carboxyl group at the α-terminal into an active ester (NHS). The reaction solution was loaded with black yeast glucan having a primary amino group at the ω-terminal (black yeast glucan-NH₂, number-average molecular weight 1,500) (749 mg), and condensation reaction (270.5 hours) of an active ester (NHS) of NHS-PLGA-OH and the primary amino group of black yeast glucan-NH₂ was performed. After the reaction, unreacted black yeast glucan-NH₂ was removed by water dialysis, and then unreacted NHS-PLGA-OH was removed by ultra-centrifugal purification to obtain a black yeast glucan-PLGA polymer 3.

A black yeast glucan-PLGA polymer 4 was obtained by the same method as for the black yeast glucan-PLGA polymer 3, using black yeast glucan-NH₂ (number-average molecular weight 1,200) and PLGA-5020 (number-average molecular weight 8,900), and a black yeast glucan-PLGA polymer 5 was obtained by the same method as for the black yeast glucan-PLGA polymer 3, using black yeast glucan-NH₂ (number-average molecular weight 2,300) and PLGA-5020 (number-average molecular weight 8,900).

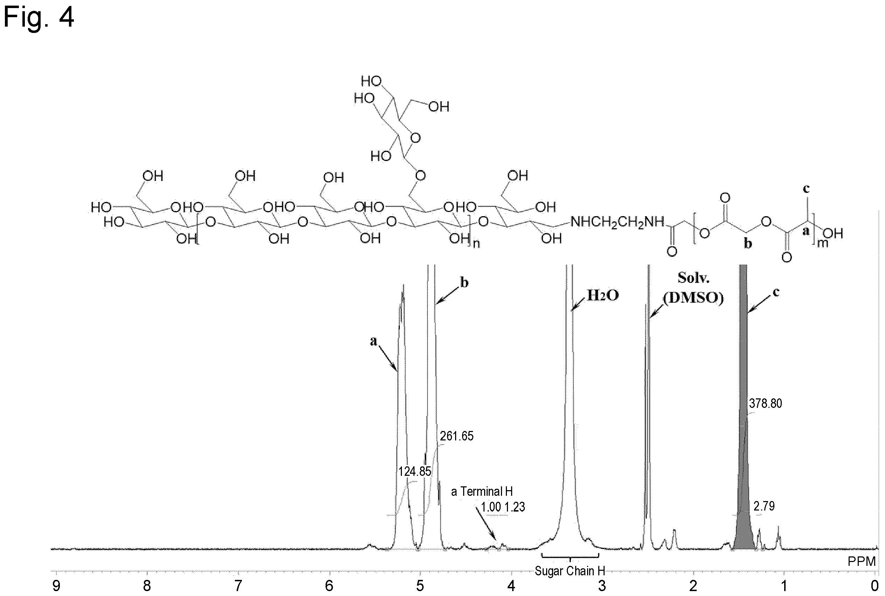

The number-average molecular weight of black yeast glucan-PLGA was determined by GPC measurement (column: TSK-gel α-5000 × 2, manufactured by Tosoh Corporation, DMF-based solvent, detector: RI, standard product: pullulan) (Table 2, Figure 3: black yeast glucan-PLGA). It was confirmed by ¹H-NMR measurement that condensation reaction progressed (Figure 4: black yeast glucan-PLGA).

**[Table 2]**

| Results of analysis on black yeast glucan-PLGA (3, 4, 5) | | | | |
|---|---|---|---|---|
| Amphiphilic polymer | Number-average molecular weight | Hydrophilic polysaccharide portion | Number-average molecular weight of hydrophilic polysaccharide | Number-average molecular weight of PLGA |
| 3 | 12200 | Black yeast glucan | 1500 | 10700 |
| 4 | 12400 | Black yeast glucan | 1200 | 11200 |
| 5 | 9900 | Black yeast glucan | 2300 | 7600 |

### (Example 3) Preparation of particles (OVA-containing dextran particle (1) and OVA-containing black yeast glucan particle (2)), by S/O/W emulsion method

The polymer powder (30 mg) of the dextran-PLGA polymer (Table 1, amphiphilic polymer 1) was dissolved in a mixture of 1.2 ml of dimethyl carbonate and 133 µl of tert-butanol to prepare a polymer solution. After dripping 0.3 ml of an aqueous 0.5% (w/v) OVA (ovalbumin, Sigma-Aldrich Co. LLC) solution into the polymer solution, the resulting mixture was stirred with a mixer (PT2100S manufactured by Polytron) at 11,000 rpm for 1 minute, to produce a W/O emulsion solution. The W/O emulsion solution was preliminarily frozen by liquid nitrogen, and then freeze-dried with a freeze-dryer (TOKYO RIKAKIKAI CO., LTD., FD-1000) at a trap cooling temperature of -45°C and a degree of vacuum of 20 Pa for 4 hours. The resulting solid content was dispersed in 3 ml of ethyl acetate to prepare a S/O suspension solution. The S/O suspension solution was dripped in 12 ml of an aqueous 1% (w/v) polyvinyl alcohol solution, and stirred with a mixer (Silverson Nippon Limited, L5M-A) at 5,000 rpm for 3 minutes to prepare a S/O/W-type emulsion solution. Ethyl acetate was removed from the S/O/W-type emulsion solution by drying in liquid, and a particle suspension liquid was obtained. The suspension liquid was transferred to a 50-ml tube, and a particle was precipitated by centrifugation at 3,000 g for 30 minutes. After the supernatant was removed, the particle was re-suspended in 50 ml of distilled water, and washed by re-precipitation of the particle with centrifugation in the above conditions. After the washing operation was repeated once more and removing the supernatant, the particle was suspended in 2.4 ml of an aqueous solution containing 5% (w/v) mannitol and 0.1% (w/v) polysorbate 80. Filtration was performed with a mesh filter (1 µm), and the suspension liquid was preliminarily frozen by liquid nitrogen and then freeze-dried with a freeze-dryer at a trap cooling temperature of -45°C and a degree of vacuum of 20 Pa for 12 hours to provide an OVA-containing dextran particle (1).

An OVA-containing black yeast glucan particle (2) was obtained by the same method as for the above OVA-containing dextran particle, using a polymer of black yeast glucan-PLGA (Table 2, amphiphilic polymer 3).

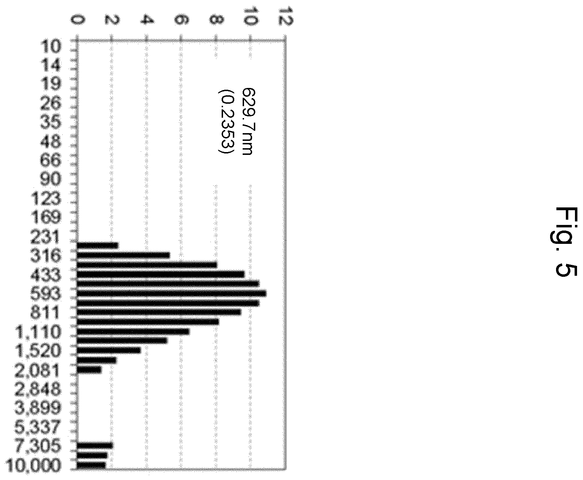

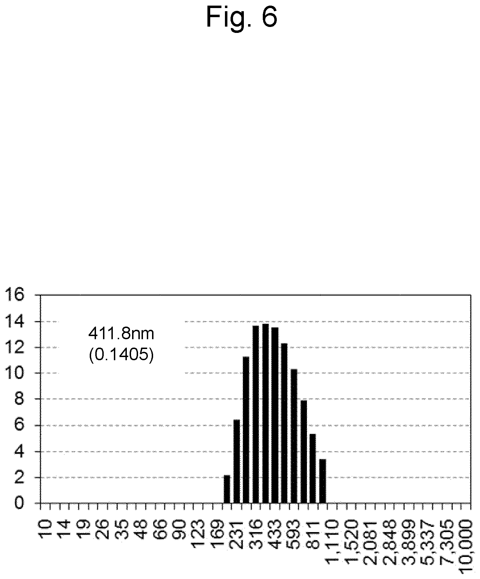

The results of the evaluation for each particle are shown in Table 3. The average particle size of the particle was calculated by a cumulant method using a dynamic light scattering apparatus (ELS-Z manufactured by OTSUKA ELECTRONICS CO., LTD.) (Table 3, Figure 5: OVA-containing dextran particle, Figure 6: OVA-containing black yeast glucan particle). The content ratio (w/w) of the OVA antigen was determined by extracting the antigen from the particle using an organic solvent, subjecting the extracted antigen to gel electrophoresis using a gel electrophoresis apparatus (TEFCO) and then staining with a colloidal CBB staining kit (TEFCO).

**[Table 3]**

| Results of analysis on particles (OVA-containing dextran particle (1) and OVA-containing black yeast glucan particle (2)) prepared by the S/O/W emulsion method | | | |
|---|---|---|---|
| Particle | Amphiphilic polymer | Average particle size (nm) | Content ratio of antigen (%) |
| (1) | Dextran-PLGA | 629.7 | 3.49 |
| (2) | Black yeast glucan-PLGA | 411.8 | 4.47 |

### (Example 4) Antitumor effect of using OVA-containing dextran particle (1) and imiquimod in combination

The OVA-containing dextran particle (1) produced in Example 3 and imiquimod were separately administered, and the *in vivo* antitumor effect in cancer-bearing mice was evaluated.

Specifically, the antitumor effect of using the OVA-containing dextran particle (1) and imiquimod in combination was studied with C57BL/6NCR mice in which OVA-expressing cancer cells were subcutaneously transplanted. A mouse lymphoma cell line EG7 (10⁶ per mouse) was subcutaneously transplanted, and 0.5 mg (except for mannitol) of the OVA-containing dextran particle (1) produced in Example 3 was subcutaneously administered to the right sides of the mice twice in total at a frequency of once a week from day 7 after transplantation, wherein the OVA-containing dextran particle (1) was suspended in a solvent of PBS and sterile water for injection so as to be isotonic. Application of imiquimod to the same mice was started at the same time as the first OVA-containing dextran particle (1). "Beselna cream 5%" (MOCHIDA PHARMACEUTICAL CO., LTD., hereinafter referred to as "imiquimod cream"), containing imiquimod as an active ingredient, was applied to the skin surface, to which the cancer cells were transplanted, for five straight days in a week. The application was not performed for the next two days, and the imiquimod cream was applied in the same manner as described above for five straight days from day 8 after the start of administration.

In a comparative control group, only the OVA-containing dextran particle (1), same as described above, was administered to cancer-bearing mice at the same dose and administration interval. In another comparative control group, only the imiquimod cream was applied to the skin surface, to which the cancer cells were transplanted, of other cancer-bearing mouse individuals at the same administration interval. Cancer-bearing mice in a non-treatment group were used as negative controls. After the start of administration, the sizes of cancers in the cancer-bearing mice were measured with calipers over time. The tumor volumes were calculated according to a standard method with a calculation formula: (Length of major axis of cancer) × (Length of minor axis of cancer)² × 0.5.

As a result of the evaluation, tumors of mice to which the administration of the OVA-containing dextran particle (1) produced in Example 3 and the application of the imiquimod cream were performed were regressed compared to those at the start of administration. On the contrary, tumors of mice to which only the OVA-containing dextran particle (1) produced in Example 3 was administered were not regressed, while delayed in growth as compared to the negative controls, and tumors of the cancer-bearing mice to which only the imiquimod cream was administered were not changed compared to the negative controls.

As a result of the present evaluation, it was demonstrated that administering the OVA-containing dextran particle and imiquimod separately in combination provides a remarkable antitumor effect compared to a case where only the OVA-containing dextran particle was administered and a case where only imiquimod was administered.

### (Example 5) Antitumor effect of using OVA-containing black yeast glucan particle (2) and imiquimod in combination

The OVA-containing black yeast glucan particle (2) produced in Example 3 and imiquimod were separately administered, and the *in vivo* antitumor effect in cancer-bearing mice was evaluated.

Specifically, the antitumor effect of using the OVA-containing black yeast glucan particle (2) and imiquimod in combination was studied with C57BL/6NCR mice in which OVA-expressing cancer cells were subcutaneously transplanted. A mouse lymphoma cell line EG7 (10⁶ per mouse) was subcutaneously transplanted, and 0.5 mg (except for mannitol) of the OVA-containing black yeast glucan particle (2) produced in Example 3 was subcutaneously administered to the right sides of mice twice in total at a frequency of once a week from day 7 after transplantation, wherein the OVA-containing black yeast glucan particle (2) was suspended in a solvent of PBS and sterile water for injection so as to be isotonic. Application of imiquimod to the same mice was started at the same time as the first OVA-containing black yeast glucan particle (2). The imiquimod cream was applied to the skin surface, to which the cancer cells were transplanted, for five straight days in a week. The application was not performed for the next two days, and the imiquimod cream was applied in the same manner as described above for five straight days from day 8 after the start of administration.

In a comparative control group, only the OVA-containing black yeast glucan particle (2), same as described above, was administered to cancer-bearing mice at the same dose and administration interval. In another comparative control group, only the imiquimod cream was applied to the skin surface to which the cancer cells were transplanted, of other cancer-bearing mouse individuals at the same administration interval. Cancer-bearing mice in a non-treatment group were used as negative controls. After the start of administration, the sizes of cancers in the cancer-bearing mice were measured with calipers over time. The tumor volumes were calculated according to a standard method with a calculation formula: (Length of major axis of cancer) × (Length of minor axis of cancer)² × 0.5.

As a result of the evaluation, tumors to which the administration of the OVA-containing black yeast glucan particle (2) produced in Example 3 and the application of the imiquimod cream were performed were regressed compared to those at the start of administration. On the contrary, tumors of mice to which only the OVA-containing black yeast glucan particle (2) produced in Example 3 was administered were not regressed, while delayed in growth compared to negative controls, and tumors of the cancer-bearing mice to which only the imiquimod cream was administered were not changed compared to negative controls.

As a result of the present evaluation, it was demonstrated that administering the OVA-containing black yeast glucan particle and imiquimod separately in combination provides a remarkable antitumor effect compared to a case where only the OVA-containing black yeast glucan particle was administered and a case where only imiquimod was administered.

### (Example 6) Antitumor effect of using OVA-containing black yeast glucan particle (2) and Poly(I:C) in combination

The OVA-containing black yeast glucan particle (2) produced in Example 3 and Poly(I:C) were separately administered, and the *in vivo* antitumor effect in cancer-bearing mice was evaluated.

Specifically, the antitumor effect of using the OVA-containing black yeast glucan particle and Poly(I:C) (Sigma-Aldrich Co. LLC, catalog number: P1530) in combination was studied with C57BL/6NCR mice in which OVA-expressing cancer cells were subcutaneously transplanted. A mouse lymphoma cell line EG7 (10⁶ per mouse) was subcutaneously transplanted, and 0.5 mg (except for mannitol) of the OVA-containing black yeast glucan particle (2) produced in Example 3 was subcutaneously administered to the right sides of mice twice in total at a frequency of once a week from day 7 after transplantation, wherein the OVA-containing dextran particle (1) was suspended in a solvent of PBS and sterile water for injection so as to be isotonic. Administration of Poly(I:C) into tumors of the same mice was started at the same time as the first OVA-containing black yeast glucan particle (2). Poly(I:C) was administered four times in total at a frequency of twice in a week at a dose of 50 µg per administration.

In a comparative control group, only the OVA-containing black yeast glucan particle (2), same as described above, was administered to cancer-bearing mice at the same dose and administration interval. In another comparative control group, only Poly(I:C) was administered into tumors of other cancer-bearing mouse individuals at the same administration interval and dose. A mixture of the OVA-containing black yeast glucan particle (2) and Poly(I:C) was administered subcutaneously to the right sides of other cancer-bearing mouse individuals at the same dose and administration interval. Cancer-bearing mice in a non-treatment group were used as negative controls. After the start of administration, the sizes of cancers in the cancer-bearing mice were measured with calipers over time. The tumor volumes were calculated according to a standard method with a calculation formula: (Length of major axis of cancer) × (Length of minor axis of cancer)² × 0.5.

As a result of the evaluation, tumors of mice in which the OVA-containing black yeast glucan particle (2) produced in Example 3 and Poly(I:C) were separately administered into such tumors were regressed compared to those at the start of administration. On the contrary, tumors of mice to which only the OVA-containing black yeast glucan particle (2) produced in Example 3 was administered were not regressed, while delayed in growth compared to negative controls, and tumors of the cancer-bearing mice to which only Poly(I:C) was administered were not changed compared to negative controls. In addition, mouse tumors to which a mixture of the OVA-containing black yeast glucan particle (2) and Poly(I:C) was administered were not regressed, while delayed in growth.

As a result of the present evaluation, it was demonstrated that the administration of the OVA-containing black yeast glucan particle and Poly(I:C) separately in combination provides a remarkable antitumor effect compared to a case where only the OVA-containing black yeast glucan particle was administered, a case where only Poly(I:C) was administered, and a case where the OVA-containing black yeast glucan particle and Poly(I:C) were mixed and administered.

### Industrial Applicability

The combination medicament of the present invention, to be used for treatment and/or prevention of cancer, which comprises a particle and an immuno-stimulating factor, wherein the particle comprises an amphiphilic polymer in which a hydrophobic segment is poly(hydroxy acid) and a hydrophilic segment is polysaccharide, and a cancer antigen, and in which the particle and the immuno-stimulating factor are separately administered, can be utilized particularly for treatment and/or prevention of infectious disease, cancer, and/or the like.

All publications, patents, and patent applications herein cited are to be herein incorporated by reference as they are.

## Claims

1. A combination medicament for use in the treatment and/or prevention of cancer, comprising a particle and an immuno-stimulating factor, wherein the particle comprises an amphiphilic polymer in which a hydrophobic segment is poly(hydroxy acid) and a hydrophilic segment is polysaccharide, and a cancer antigen, and
wherein the use is **characterized in that** administration of the particle and administration of the immuno-stimulating factor are performed separately.

2. The medicament according to claim 1, wherein the use is **characterized in that** administration of the particle and administration of the immuno-stimulating factor are performed separately by administration methods different from each other.

3. The medicament according to claim 1 or 2, wherein the immuno-stimulating factor is a ligand or agonist binding to a Toll-like receptor (TLR), a NOD-like receptor (NLR), a RIG-like receptor, a C-type lectin receptor (CLR) or a stimulator of interferon gene (STING).

4. The medicament according to claim 3, wherein the ligand or agonist binding to the Toll-like receptor (TLR) is a ligand or agonist binding to TLR2, TLR3, TLR4, TLR5, TLR7, TLR8, TLR9, or TLR11.

5. The medicament according to claim 4, wherein the ligand or agonist binding to the Toll-like receptor (TLR) is any of the following (i) to (vii):
(i) a ligand or agonist binding to TLR2 selected from the group consisting of peptidoglycan, lipoprotein, lipopolysaccharide and zymosan;
(ii) a ligand or agonist binding to TLR3 selected from the group consisting of poly(I:C) and poly(A:U);
(iii) a ligand or agonist binding to TLR4 selected from the group consisting of lipopolysaccharide (LPS), HSP60, RS09, and MPLA;
(iv) flagellin as a ligand or agonist binding to TLR5;
(v) a ligand or agonist binding to TLR7 or 8 selected from the group consisting of an imidazoquinoline compound and single-strand RNA;
(vi) a ligand or agonist binding to TLR9 selected from the group consisting of bacterial DNA, unmethylated CpG DNA, hemozorin, ODN1585, ODN1668, and ODN1826; and
(vii) a ligand or agonist binding to TLR11 selected from the group consisting of profilin and uropathogenic bacteria.

6. The medicament according to claim 5, wherein the imidazoquinoline compound is imiquimod.

7. The medicament according to any one of claims 1 to 6, wherein administration of the immuno-stimulating factor is administration to a local tumor area.

8. The medicament according to any one of claims 1 to 7, wherein the polysaccharide is dextran, β-glucan, mannan, chitin, chitosan, gellan gum, alginic acid, hyaluronic acid or pullulan.

9. The medicament according to claim 8, wherein the β-glucan is a polymer of glucose linked by one or more β-1,3 bonds and/or one or more β-1,6 bonds.

10. The medicament according to claim 8 or 9, wherein the β-glucan is black yeast glucan, curdlan, pachyman, laminaran, lichenan, schizophyllan, lentinan, scleroglucan, or pachymaran.

11. The medicament according to any one of claims 1 to 10, wherein the poly(hydroxy acid) is poly(lactic-co-glycolic acid), polylactic acid, or polyglycolic acid.

12. The medicament according to any one of claims 1 to 11, wherein a number-average molecular weight of the amphiphilic polymer is 500 to 100,000.

13. The medicament according to any one of claims 1 to 12, wherein an average particle size of the particle is 0.1 to 50 µm.

14. The medicament according to any one of claims 1 to 13, wherein the cancer is basal cell cancer, Paget's disease, skin cancer, breast cancer, kidney cancer, pancreatic cancer, colon cancer, lung cancer, brain tumor, gastric cancer, uterus cancer, ovarian cancer, prostate cancer, urinary bladder cancer, esophageal cancer, leukemia, lymphoma, liver cancer, gallbladder cancer, sarcoma, mastocytoma, adrenal cortex cancer, Ewing's tumor, Hodgkin's lymphoma, mesothelioma, multiple myeloma, testicle cancer, thyroid cancer, or head and neck cancer.

15. A method for treating and/or preventing cancer, comprising separately administering: a particle comprising an amphiphilic polymer in which a hydrophobic segment is poly(hydroxy acid) and a hydrophilic segment is polysaccharide, and a cancer antigen; and an immuno-stimulating factor.

16. The method according to claim 15, wherein administration of the particle and administration of the immuno-stimulating factor are separately performed by administration methods different from each other.
